# EUROPEAN PATENT APPLICATION

(11) **EP 3 760 645 A1**
(43) Date of publication of application: **06.01.2021**
(21) Application number: 19188684.5
(22) Date of filing: 26.07.2019
(51) Int. Cl.: C07K 16/34, C12N 5/078, G01N 33/80

(54) **ANALYSIS FOR BLOOD GROUP ANTIGEN DACY**

(30) Priority: 02.07.2019 EP 19183971
(71) Applicant: imusyn GmbH & Co. KG, 30625 Hannover (DE)
(72) Inventor: Schneeweiß, Clemens, 30625 Hannover (DE); Grüger, Daniela, 30625 Hannover (DE); Beyer, Anja, 30625 Hannover (DE); Habicht, Cora, 30625 Hannover (DE); Skaik, Younis, 30625 Hannover (DE)
(74) Representative: Taruttis, Stefan Georg

(57) **Abstract**

The invention provides a newly identified blood group antigen (BGA), analytical processes for identifying the presence of the nucleotide sequence encoding this BGA and analytical processes for identifying this BGA protein and an analytical process for identifying the presence of antibody directed against this BGA in a blood serum sample. The invention provides two allelic variants of SEQ ID NO: 1, also termed Dacy. These variants have been found to be BGA, which are epitopes that are recognized by serum antibodies, the BGA, or epitopes. These epitopes have been found to be functional, e.g. found to be recognized by serum antibodies which also recognize full-length CR1 protein in soluble form, e.g. lacking the transmembrane domain, as well as natural full-length CR1 membrane-bound on RBC or RBC membrane fragments.

## Description

Analysis for Blood Group Antigen Dacy. The present invention relates to a newly identified blood group antigen (BGA), to analytical processes for identifying the presence of the nucleotide sequence encoding this BGA and/or to analytical processes for identifying this BGA protein, e.g using serum pre-determined to contain antibody specific for the newly identified BGA, and/or an analytical process for identifying the presence of antibody directed against this BGA in a blood serum sample, and to the use of this BGA protein as an antigen, e.g. in the analytical process. The newly identified BGA is presently named Dacy. It was found that Dacy occurs in two allelic variants, which are named Dacy(a) and Dacy(b).

Further, the invention relates to isolated mammalian cell membranes, e.g. whole cells, e.g. red blood cells, or isolated cell membranes, e.g. of red blood cells, which are pre-determined to comprise only one of Dacy(a) and Dacy(b), for use as an antigen in an analytical process for determining antibodies in a blood serum sample. Accordingly, the invention also relates to an analytical process for determining antibodies in a blood serum sample, the process comprising the step of contacting a serum sample with whole cells, e.g. red blood cells, or with cell membranes, e.g. of red blood cells, which are pre-determined to comprise one of Dacy(a) and Dacy(b).

It is generally known that blood serum (herein also termed serum) may contain antibodies directed against BGA (anti-BGA-antibody) which are not naturally present in the organism, e.g. in a human, but to which the human has been exposed, e.g. by blood transfusion. Anti-BGA-antibodies and/or presence of BGA are analyzed prior to transplantation, e.g. prior to transplantation of cells, especially prior to blood transfusion or transplantation of a solid tissue or organ, in order to prevent immune reactions between the serum anti-BGA-antibodies and cells.

### State of the art

It is generally known to determine BGA on red blood cells (RBC) by contacting with a serum that is pre-determined to contain antibody specific for certain BGA, wherein agglutination of the RBC in presence of the serum indicates presence of the certain BGA. Serum with pre-determined specificities can be identified by their reaction with RBC of known BGA, e.g. pre-determined by DNA analysis or immunologically.

Moulds et al., Blood, 2879-2885 (2001), describe analyses of BGA of the Knops (KN) group, which includes allelic variants Knops a and b (Kn(a), Kn(b)), allelic variants of McCoy (McC(a/b), Swain-Langley (Sl(a)) and Villien (Vil), in malaria-infected red blood cells (RBC). These members of the Kn group of BGA are located in the domain LHR-D of CR1 (human complement receptor 1).

Moulds and Rowe, Transfusion 517-520 (1996), describe that cloned soluble CR1 protein could be used to neutralize serum antibodies directed against the Knops (Kn) group.

Furtado et al., J. Mol. Biol 102-118 (2008), describe the solution structure of the 30 short complement regulator (SCR) domains, which can also be named Sushi1 to Sushi30, that make up CR1 (CD35). As a model protein, the soluble CR1 protein lacked the C-terminal transmembrane and cytoplasmic domains.

Tamasauskas et al., Transfusion 1397-1404 (2001), describe analyses of BGA in relation to binding of malaria ligands during the course of malaria infections.

### Object of the invention

The object of the invention is to provide the antigen of red blood cells (RBC) that is responsible for an antibody reaction from blood sera that was previously not be attributed to known BGA. When analysing blood sera for presence of antibodies reacting with RBC, it was found that for some sera, antibody reactions could not be explained fully. Accordingly, it is also an object of the invention to provide for an analytical process for determining the antigen that can cause serum reactions not caused by a known antigen. It is a further object of the invention to provide an analytical process for detecting and identifying serum antibody having a specificity that was previously not attributed to be specific for a known BGA.

### Description of the invention

Surprisingly, a polymorphism H1208R located in the SCR domain 19 (Sushi19) of CR1 (SEQ ID NO: 1) was determined, and immunologic analyses showed that blood serum reactions could be attributed to red blood cells carrying one of the two allelic variants of this polymorphism. Accordingly, the invention achieves the objects by the features of the claims, especially by providing two allelic variants of H1208R (numbering of full-length amino acid sequence of CR1, SEQ ID NO: 1) of CR1, which variants herein are also termed Dacy, Dacy(a) for the H1208 (Histidine in amino acid position No. 1208 of SEQ ID NO: 1) variant, Dacy(b) for the R1208 (Arginine in amino acid position No. 1208 of SEQ ID NO: 1) variant. These variants have been found to be BGA, which are epitopes that are recognized by serum antibodies, the BGA, or epitopes, containing one of H1208 or R1208 of SEQ ID NO: 1. These epitopes have been found to be functional, e.g. found to be recognized by serum antibodies which also recognize full-length CR1 protein in soluble form, e.g. lacking the transmembrane domain, as well as natural full-length CR1 membrane-bound on RBC or RBC membrane fragments.

The allelic variants of the polymorphism of the invention can be contained in a protein comprising or consisting of the SCR domain 19 (Sushi19), the C3b and/or C4b, or the LHR-C domain, or full-length CR1 (SEQ ID NO: 1), optionally lacking the signal peptide (amino acids 1..41 of SEQ ID NO: 1) and/or the transmembrane domain (amino acids 1972..1996 of SEQ ID NO: 1) and/or the cytoplasmic domain (amino acids 1997..2039 of SEQ ID NO: 1). For example, the protein comprising at least one of the allelic variants of Dacy can be devoid of other BGA epitopes, e.g. the protein can be devoid of the CR1 domains LHR-A, LHR-B and LHR-D, and optionally additionally be devoid of any one or more or all of SCR 15, SCR 16, SCR 17, SCR 18, SCR 20 and SCR 21. Generally optionally, the protein can comprise additional amino acid sequences which do not contain a BGA epitope.

Further, the invention provides cells and cell membrane fragments, which contain one of the allelic variants of Dacy. Dacy, respectively its allelic variants and its function as a BGA eptitope, have been identified by the present invention. The allelic variants of Dacy can be contained in, e.g. presented on, natural human cells, e.g. RBC, or on membrane fragments of such cells, e.g. ghosts, wherein the specific allele of Dacy of a cell is pre-determined. Preferably, in such cells or their membrane fragments, the presence of other known BGA is also pre-determined.

Accordingly, in cells, which preferably are RBC, or in membrane fragments prepared from these cells, the presence of the allele of Dacy is pre-determined, e.g. by serologic analysis or DNA sequencing, and these cells or membrane fragments prepared from these cells are characterized in that the allele of Dacy is pre-determined and thus known to the user and can positively be used in interpreting analytical results. The interpretation of analytical results can be made in knowledge of the pre-determined presence of the allele Dacy(a) and/or Dacy(b) on the cells or membrane fragments, and therefore analytical results of serum testing for anti-BGA-antibodies can be interpreted with greater accuracy. Cells and membrane fragments according to the invention which are pre-determined in respect of their specific Dacy allele can be used in analytical processes which are agglutination assays, e.g. in the Coombs gel assay, or in assays using immobilization of the cell or membrane fragment and detection of the binding of antibodies from a serum sample, e.g. in an ELISA format using the cell or membrane fragment as the immobilized antigen, or in a solid phase adherence assay with indicator RBC, e.g. using the capture assay available from Immucor, USA, e.g. as described at http://www.immucor.com/en-us/Products/Documents/Immucor_Capture-Brochure_US_Web.pdf.

Generally in the field of art, membrane fragments of cells, especially of RBC, are also referred to as ghosts, due to their lack of colour.

Optionally, the invention provides proteins containing one or both of the allelic variants of Dacy, which proteins, e.g. in the case of a protein containing both allelic variants as e.g. a synthetic or non-natural protein, can be produced by expression of a nucleic acid sequence encoding the protein, e.g. in a mammalian cell culture. Preferably, the protein containing one allelic variant of Dacy, or which protein in case of a protein which is synthetic or expressed from a synthetic coding DNA sequence can contain both allelic variants of Dacy, is for use in a process for testing sera for presence of anti-BGA-antibodies.

Further, the invention provides a process for analysing a serum sample for the presence of antibody directed against one or both of the allelic variants of Dacy, and respectively a process for analysing a cell-containing sample, e.g. blood cells or tissue, for the allelic variants of Dacy, e.g. using a serum that was pre-determined to contain one of anti-Dacy(a) or anti-Dacy(b) antibody.

It was found now that prior to the present invention, analytical processes for determining anti-BGA-antibodies in a serum sample could yield a positive reaction of RBC or their membrane fragments, which positive reaction could not be attributed to one of the BGA molecules that were known to be present on the RBC or membrane fragment. The invention now provides mammalian cells, e.g. red blood cells (RBC), or membranes of these, which are pre-determined to comprise either Dacy(a) or Dacy(b). Such cells or membrane fragments are suitable for use in an analytical process for determining the presence of antibodies directed against blood group antigens and such cells and membrane fragments allow an analytical process for determining the presence of antibodies directed against blood group antigens, including the identification or attribution of a serum antibody reaction to be specific for Dacy(a) and/or Dacy(b), respectively to indicate presence of a serum antibody specific for Dacy(a) and/or Dacy(b).

The pre-determination of the allelic variant, i.e. Dacy(a) or Dacy(b), present in a batch of cells can be by DNA analysis, e.g. sequencing or sequence specific priming analysis of DNA, or by immunologic analysis of the batch of cells using serum known to contain antibodies specific for Dacy(a) or Dacy(b).

In a further embodiment, the invention provides a process for determination of the allelic variant of Dacy by DNA analysis of DNA obtained from a sample, e.g. from blood, wherein the process comprises or consists of the step of determining the DNA section encoding the alleles of Dacy. DNA analysis can e.g. be by sequencing or sequence specific priming analysis of DNA.

In a further embodiment, the invention provides a protein comprising one or both of the allelic variants of Dacy for inhibiting antibodies directed against Dacy, e.g. in a process for analysis of anti-BGA antibodies in a serum sample. In this embodiment, the protein containing at least one allelic variant of Dacy can be used for blocking specific antibodies in order to specifically prevent these antibodies to cause a positive indication of antibody-antigen reactions, e.g. for allowing the detection of other antibodies reacting with their antigen. A protein comprising one or both allelic variants of Dacy could be expressed in mammalian cell culture, in other eukaryotic cells, e.g. in yeast or fungal or insect cells, or in prokaryotic cells, from a synthetic DNA coding sequence. In this embodiment, a protein comprising one or both allelic variants of Dacy is added to a serum sample to be tested for its content of antibodies, followed by contacting the serum sample with additional antigen, e.g. isolated BGA and/or membranes of RBC, and detecting binding of antibody to the additional antigen, wherein antibodies of the serum sample directed against one or both allelic variants of Dacy are blocked and e.g. do not cause a detectable reaction.

It was found that the alleles of Dacy are antithetic.

Further, the invention provides the use of a protein as described herein, the protein containing the epitope of Dacy(a) and/or of Dacy(b), preferably only one or both of these epitopes, in a process for producing antibody specific for the epitope of Dacy(a) and/or of Dacy(b), preferably only one or both of these epitopes. Processes for producing antibody, monoclonal or polyclonal, using immunization of an animal and/or in vitro processes, are known.

In the figures,
- Fig. 1 shows relative concentrations of human antibodies in an ELISA,
- Fig. 2 shows the result of a gel-card agglutination analysis,
- Fig. 3 shows sequencing data of a portion of the coding sequence of Dacy originating from different blood donors, and

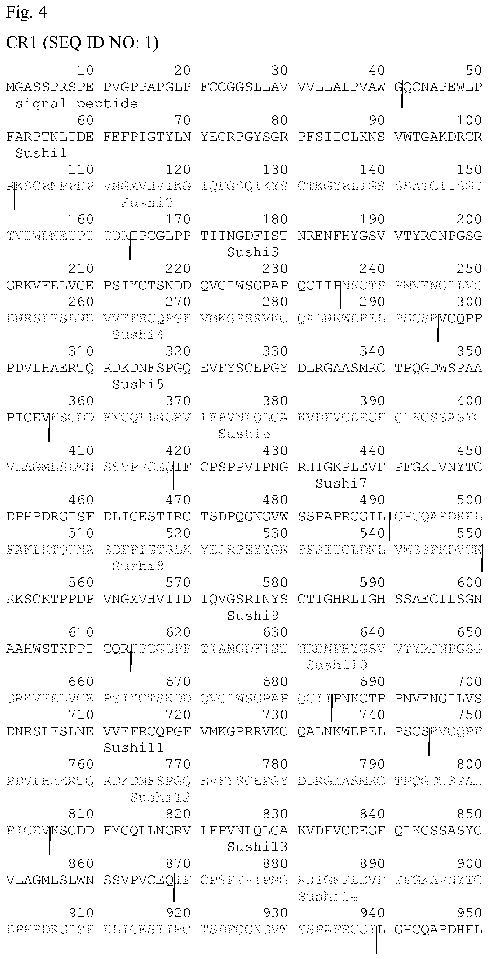

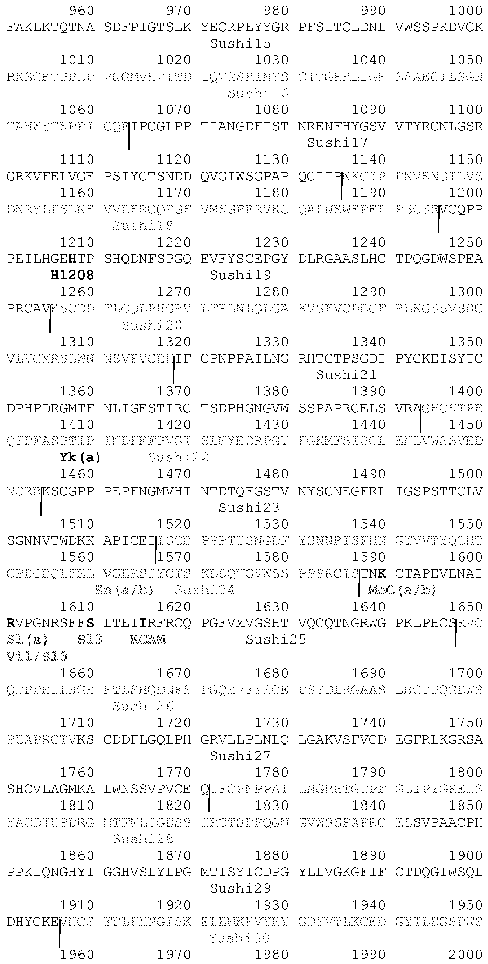

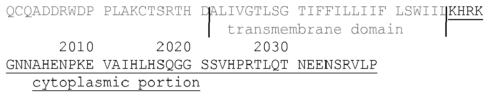

- Fig. 4 shows the amino acid sequence of CR1 (SEQ ID NO: 1), wherein vertical lines indicate the limits of individual domains, e.g. of the Sushi domains.

### Example 1: Production of a protein containing one or both alleles of Dacy as the only BGA

The DNA sequence encoding the LHR-C (Sushi15 to Sushi21, amino acids 940..1393 of SEQ ID NO: 1) of CR1 was synthesized commercially (Thermo Fisher Scientific, MA, USA) and ligated into a modified lentiviral expression vector pRRL-PPT-SFFV, containing a coding sequence for a signal peptide upstream of the coding sequence for LHR-C of CR1 and containing the coding sequences for two protein tags (V5 and polyhistidine) downstream of the coding sequence for LHR-C of CR1. Preferably, the coding sequence was codon-optimized for HEK293 cells. In the coding sequence for LHR-C of CR1, the H1208R variant was generated by site-directed mutagenesis using two overlapping primers containing the mutation and the QuickChange Lightning Kit (obtained from Agilent, CA, USA). Separately, each of the lentiviral expression vectors containing a coding sequence for one of the alleles were separately transfected into HEK293T cells together with two helper plasmids (pMD2G and psPAX2) for the production of lentiviral particles. The lentiviral particles were harvested for the cell culture supernatant and used to transduce HEK293 cells. Stable integration of the expression cassette containing the LHR-C coding sequence into the genome of cells was controlled by measuring the concentration of the secreted LHR-C protein in cell culture supernatant.

From separate cultures, the cell culture supernatant was collected and buffered into PBS (7 mM NaH₂PO₄, 150 mM NaCl, pH 7.4). LHR-C protein was bound to an immobilized metal affinity chromatography (IMAC) column via the histidine tag, the column was washed, and bound LHR-C protein was eluted with imidazol, to yield Dacy(a) and Dacy(b) in separate preparations. The eluates were buffered into PBS, optionally containing a preserving agent, e.g. ProClin 300 (obtainable from Sigma Aldrich), adjusted to 400 to 500 ng/mL, and stored at 4°C.

### Example 2: Detection of anti-Dacy antibody in serum samples

For immobilization, the separate proteins containing Dacy(a) or Dacy(b) produced according to Example 1 were added in an amount of 100 ng protein per well (microtiter plate C96 Maxisorp, Thermo Fisher Scientific) in 100 µL PBS and incubated overnight at 4°C. Subsequent blocking of free binding sites of the wells was with PSTB (7 mM NaH₂PO₄, 150 mM NaCl, pH 7.4, 0,05 % v/v Tween 20,2 % w/v bovine serum albumin) for 1 h at room temperature. Unbound protein was removed by washing twice with PBST (7 mM NaH₂PO₄, 150 mM NaCl, pH 7.4, 0,05 % v/v Tween 20).

Serum samples, designated A to E, suspected of containing binding antibodies, were diluted 1:2 in PBST and added to washed wells at 100 µL and incubated for 1 h at 37°C. After this incubation, the plate was washed 4 times with PBST. For detection of bound antibody, 100 µL PIKVA (1:20000 anti-human IgG-HRP antibody conjugate, MCA647P, Bio-Rad, CA, USA, 50% v/v fetal calf serum, 1.06 %v/v tris(hydroxymethyl)amino methane, 0.5 % w/v PVP-10 (polyvinyl pyrrolidone, average mol. Weight 10 000), 0.45% w/v NaCl, 0.1 % w/v Ca-propionate, optionally 0.2 % v/v ProClin 300, 0.05 % w/v 8-anilino-1-naphthalene sulfonic acid (ANS), 0.025 % penicillin, 0.025 % streptomycin, adjusted to pH 7.35) was added and incubated for 1 h at 37°C. Then, the plate was washed 6 times with PBST and then 100 µL TMB ONE (obtained from Kem-En-Tec Diagnostics, Denmark) was added for colour development, then 100 µL stop solution (5 %v/v H₂SO₄) was added. Absorbance was measured in a microtiter plate reader (Powerwave 320, Bio-Tek) at 450 nm (OD_{450 nm}).

In separate wells, isolated BGA protein LHR-D, containing the epitopes for Yk(a+), Kn(a), McC(a), S13 and KCAM+ (obtainable from imusyn GmbH & Co. KG), or isolated Lu(b) (obtainable from imusyn GmbH & Co. KG) or, as a negative control, PBSO (PBS without protein) were immobilized.

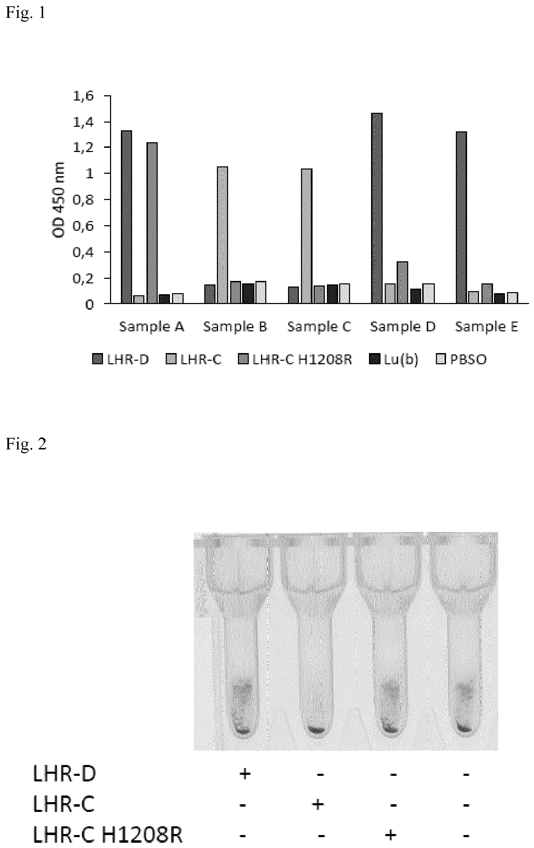

The results are shown in Fig. 1, wherein the OD_{450 nm} is given on the Y-axis, and for each sample A to E, from left to right, the first column is for immobilized LHR-D, the second column is for immobilized LHR-C H1208 (Dacy(a)), the third column is for immobilized LHR-C R1208 (Dacy(b)), the fourth column is for immobilized Lu(b), and the fifth column is PBSO (PBS buffer without protein) as negative control. The results show that either no anti-Dacy antibody is contained in the serum or only one of anti-Dacy(a) antibody or anti-Dacy(b) antibody is present. In Sample A, only anti-LHR-D antibody and anti-Dacy(b) antibody are detected, and anti-Dacy(a) and anti-Lu(b) show background level signals (PBSO). In Sample B and in Sample C, only anti-Dacy(a) is detected, the other proteins are at background signal level. In Sample D anti-LHR-D antibody is clearly detected, and a very weak signal for anti-Dacy(b) antibody, the other proteins give background signal levels only. In Sample E, only anti-LHR-D antibody is clearly detected, the other proteins give background signal levels only.

### Example 3: Blocking of anti-Dacy serum antibodies in a serum sample

In order to suppress a signal when analysing a serum sample for anti-BGA antibodies, a protein containing the epitope of Dacy(a) or of Dacy(b) can be added to a serum sample prior to contacting the serum sample with other BGA. As an example for blocking anti-Dacy antibody in a serum sample, 1 µg of one of the following purified proteins was added to separate aliquots of 25 µL of one serum sample suspected of containing anti-Dacy(a) antibody: LHR-D, LHR-C H1208 (Dacy(a)), LHR-C R1208 (Dacy(b)), or no protein (negative control).

Following addition of the purified protein, the serum aliquots were incubated for 30 min at room temperature. Subsequently, the samples were analysed in a gel agglutination assay using the addition of RBC to the serum samples and incubation for 15 min at 37°C. The gel agglutination assay was performed as a LISS/Coombs gel card assay obtained from Bio-Rad according to the manufacturer's protocol.

The results are depicted in Fig. 2, wherein the presence of the LHR-D, LHR-C H1208 (Dacy(a)), or LHR-C R1208 (Dacy(b)) protein added is indicated by +. The first (left) gel analysis shows that addition of LHR-D protein only prior to the agglutination assay results in medium intensity agglutination of the RBC, the second gel analysis shows no agglutination in presence of LHR-C H1208 (Dacy(a)), the third gel analysis shows medium intensity agglutination of the RBC in the presence of LHR-C R1208 (Dacy(b)), and the negative control (no protein added) shows shows medium intensity agglutination.

This results shows that in this serum sample, addition of LHR-C H1208 (Dacy(a)) abolished agglutination, whereas addition of LHR-C R1208 (Dacy(b)) did not significantly change agglutination compared to the negative control. Also, addition of LHR-D did not significantly change agglutination. As addition of LHR-C H1208 (Dacy(a)) abolished agglutination, this shows that the serum sample contained anti-LHR-C H1208 (anti-Dacy(a)) antibody, but no anti-LHR-C R1208 (anti-Dacy(b)) antibody.

### Example 4: Detection of presence of the Dacy allele by DNA sequencing

As an alternative to analysis of the presence of Dacy(a) or Dacy(b) protein or no Dacy protein on cells, following removal of RBC, the total DNA from nucleated blood cells of a sample was isolated and sequenced and the coding sequence for amino acid No. 1208 of SEQ ID NO: 1 was determined.

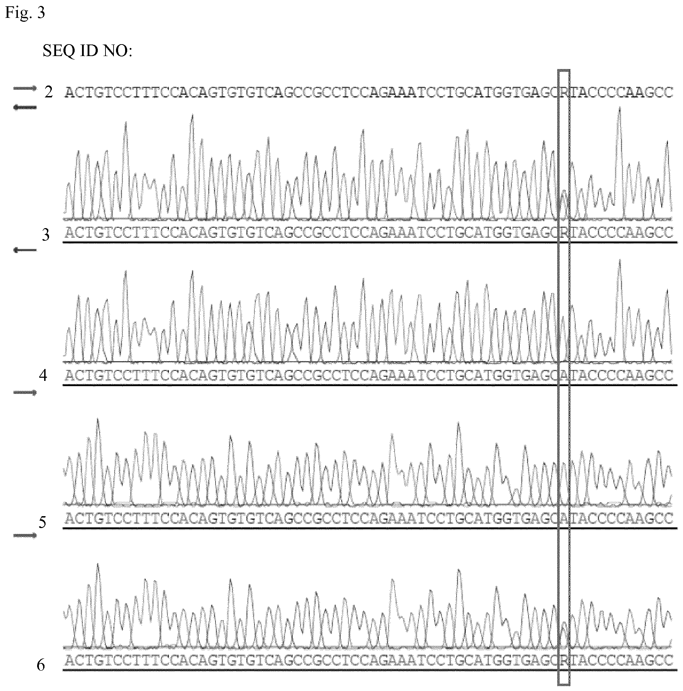

It was found that two samples were homozygous Dacy(a), while two samples were heterozygous Dacy(a) and Dacy(b) (Fig. 3). The sequences of Fig. 3 from top to bottom are SEQ ID NO: 2, SEQ ID NO: 3, SEQ ID NO: 4, SEQ ID NO: 5, SEQ ID NO: 6.

### Example 5: Immunologic detection of Dacy allele using serum

Serum containing antibody specific for Dacy(a) was pre-determined from a variety of blood serum samples obtained from patients that had previously received blood transfusion. For pre-determination, the serum samples were analysed as described in Example 2.

Each of the sera was used in a Coombs gel agglutination assay with different samples containing RBC. It was found that the sera were suitable to identify Dacy(a) on the RBC samples.

## Claims

1. Mammalian cell or mammalian cell membrane for use in an analytical process for determination of presence of serum antibody in a serum sample, **characterized in that** the mammalian cell or mammalian cell membrane is pre-determined to comprise a protein having one of the epitopes containing H1208 (Dacy(a)) or R1208 (Dacy(b)) of the amino acid sequence of CR1 (SEQ ID NO: 1).

2. Mammalian cell or mammalian cell membrane according to claim 1, **characterized in that** the amino acid sequence of CR1 (SEQ ID NO: 1) containing H1208 (Dacy(a)) or R1208 (Dacy(b)) is pre-determined serologically or by DNA analysis of DNA isolated from the mammal, or from a cultivated cell derived from such mammal, from which the cell or cell membrane originates.

3. Mammalian cell or mammalian cell membrane according to one of claims 1 to 2, **characterized in that** the protein is comprised in membrane-bound CR1 protein.

4. Protein for use in the analysis of serum antibodies, **characterized in that** the protein comprises the amino acids No. 1196..1255 of SEQ ID NO: 1 (Sushi19 domain).

5. Protein according to claim 4, **characterized in that** it is free from at least one or all of the blood group antigens (BGA) Yk(a) and Yk(a-), Kn(a) and Kn(b), McC(a) and McC(b), Sl(a) and Vil, S13 and KCAM.

6. Protein according to one of claims 4 to 5, **characterized in that** it is free from at least one of the domains Sushi22 (amino acids No. 1394..1454 of SEQ ID NO: 1), Sushi24 (amino acids No. 1517..1587 of SEQ ID NO: 1) and/or Sushi25 (amino acids No. 1588..1647 of SEQ ID NO: 1) and/or Sushi28 (amino acids No. 1772..1842 of SEQ ID NO: 1) and/or Sushi29 (amino acids No. 1843..1906 of SEQ ID NO: 1).

7. Protein according to one of claims 4 to 6, **characterized in that** the protein among sequences originating from CR1 (SEQ ID NO: 1) contains only the Sushi19 domain (amino acids No. 1196..1255 of SEQ ID NO: 1).

8. Analytical process for the determination of the presence of serum antibody in a serum sample, which antibody is directed against a blood group antigen, **characterized by** adding to the serum sample a mammalian cell or mammalian cell membrane according to one of claims 1 to 3, and/or adding to the serum sample a protein according to one of claims 4 to 7, followed by determining the binding of antibody from the serum sample to the mammalian cell or mammalian cell membrane and/or to the protein according to one of claims 4 to 7.

9. Analytical process according to claim 8, **characterized in that** prior to contacting the serum sample with the mammalian cell or mammalian cell membrane and/or with at least one isolated BGA, the serum sample is mixed with a protein according to one of claims 4 to 7.

10. Analytical process according to one of claims 8 to 9, **characterized in that** the protein only contains the blood group antigens contained in the group consisting of H1208 (Dacy(a)) and R1208 (Dacy(b)) of the amino acid sequence of CR1 (SEQ ID NO: 1), Yk(a) and Yk(b), Kn(a) and Kn(b), McC(a/b), Sl(a), Sl(b), S13 and KCAM.

11. Analytical process according to one of claims 8 to 10, **characterized in that** the protein only contains the blood group antigens contained in amino acids No. 1196..1906 of SEQ ID NO: 1 (domains Sushi19 to Sushi29).

12. Analytical process according to one of claims 8 to 11, wherein the serum sample contains antibody directed against one of the alleles of H1208 (Dacy(a)) or R1208 (Dacy(b)) of SEQ ID NO: 1.

13. Analytical process for determination of the allele of H1208 (Dacy(a)) or R1208 (Dacy(b)) of SEQ ID NO: 1 in a biological sample, comprising analysing DNA of the sample and determining the codon coding for amino acid No. 1208 of SEQ ID NO: 1.

14. Analytical process for the determination of the presence of a blood group antigen (BGA) having one of the epitopes containing H1208 (Dacy(a)) or R1208 (Dacy(b)) of the amino acid sequence of CR1 (SEQ ID NO: 1) in a blood sample, comprising contacting cells of the blood sample separately with a serum pre-determined to contain antibody specific for H1208 of SEQ ID NO: 1 or with a serum pre-determined to contain antibody specific for R1208 of SEQ ID NO: 1.

15. Analytical process according to one of claims 8 to 14, wherein the result of the analytical process is communicated to the human from whom the sample originated.


SEQUENCE LISTING

<110> Imusyn GmbH & Co. KG

<120> Analysis for Blood Group Antigen Dacy

<130> I1033EP

<150> EP19183971.1
<151> 2019-07-02

<160> 6

<170> BiSSAP 1.3.6

<210> 1
<211> 2039
<212> PRT
<213> Homo sapiens

<220>
<221> SIGNAL
<222> 1..41
<223> signal peptide

<220>
<221> CHAIN
<222> 42..101
<223> Sushi1

<220>
<221> CHAIN
<222> 102..163
<223> Sushi2

<220>
<221> CHAIN
<222> 164..235
<223> Sushi3

<220>
<221> CHAIN
<222> 236..295
<223> Sushi4

<220>
<221> CHAIN
<222> 296..355
<223> Sushi5

<220>
<221> CHAIN
<222> 356..418
<223> Sushi6

<220>
<221> CHAIN
<222> 419..490
<223> Sushi7

<220>
<221> CHAIN

<222> 491..551
<223> Sushi8

<220>
<221> CHAIN
<222> 552..613
<223> Sushi9

<220>
<221> CHAIN
<222> 614..684
<223> Sushi10

<220>
<221> CHAIN
<222> 685..744
<223> Sushi11

<220>
<221> CHAIN
<222> 745..805
<223> Sushi12

<220>
<221> CHAIN
<222> 806..868
<223> Sushi13

<220>
<221> CHAIN
<222> 869..939
<223> Sushi14

<220>
<221> CHAIN
<222> 940..1001
<223> Sushi15

<220>
<221> CHAIN
<222> 1002..1063
<223> Sushi16

<220>
<221> CHAIN
<222> 1064..1135
<223> Sushi17

<220>
<221> CHAIN
<222> 1136..1195
<223> Sushi18

<220>
<221> CHAIN
<222> 1196..1255
<223> Sushi19

<220>
<221> SITE
<222> 1208
<223> H1208

<220>
<221> CHAIN
<222> 1256..1318
<223> Sushi20

<220>
<221> CHAIN
<222> 1319..1393
<223> Sushi21

<220>
<221> CHAIN
<222> 1394..1454
<223> Sushi22

<220>
<221> SITE
<222> 1408
<223> Yk(a)

<220>
<221> CHAIN
<222> 1455..1516
<223> Sushi23

<220>
<221> CHAIN
<222> 1517..1587
<223> Sushi24

<220>
<221> SITE
<222> 1561
<223> Kn(a/b)

<220>
<221> CHAIN
<222> 1588..1647
<223> Sushi25

<220>
<221> SITE
<222> 1590
<223> McC(a/b)

<220>
<221> SITE
<222> 1601
<223> Sl(a) Vil(S13

<220>
<221> SITE
<222> 1610
<223> S13

<220>
<221> SITE
<222> 1615
<223> KCAM

<220>
<221> CHAIN
<222> 1648..1708

<223> Sushi26

<220>
<221> CHAIN
<222> 1709..1771
<223> Sushi27

<220>
<221> CHAIN
<222> 1772..1842
<223> Sushi28

<220>
<221> CHAIN
<222> 1843..1906
<223> Sushi29

<220>
<221> CHAIN
<222> 1907..1971
<223> Sushi30

<220>
<221> TRANSMEM
<222> 1972..1996

<220>
<221> CHAIN
<222> 1997..2039
<223> cytoplasmic portion

<400> 1
Met Gly Ala Ser Ser Pro Arg Ser Pro Glu Pro Val Gly Pro Pro Ala
1               5                   10                  15
Pro Gly Leu Pro Phe Cys Cys Gly Gly Ser Leu Leu Ala Val Val Val
            20                  25                  30
Leu Leu Ala Leu Pro Val Ala Trp Gly Gln Cys Asn Ala Pro Glu Trp
        35                  40                  45
Leu Pro Phe Ala Arg Pro Thr Asn Leu Thr Asp Glu Phe Glu Phe Pro
    50                  55                  60
Ile Gly Thr Tyr Leu Asn Tyr Glu Cys Arg Pro Gly Tyr Ser Gly Arg
65                  70                  75                  80
Pro Phe Ser Ile Ile Cys Leu Lys Asn Ser Val Trp Thr Gly Ala Lys
                85                  90                  95
Asp Arg Cys Arg Arg Lys Ser Cys Arg Asn Pro Pro Asp Pro Val Asn
            100                 105                 110
Gly Met Val His Val Ile Lys Gly Ile Gln Phe Gly Ser Gln Ile Lys
        115                 120                 125
Tyr Ser Cys Thr Lys Gly Tyr Arg Leu Ile Gly Ser Ser Ser Ala Thr
    130                 135                 140
Cys Ile Ile Ser Gly Asp Thr Val Ile Trp Asp Asn Glu Thr Pro Ile
145                 150                 155                 160
Cys Asp Arg Ile Pro Cys Gly Leu Pro Pro Thr Ile Thr Asn Gly Asp
                165                 170                 175
Phe Ile Ser Thr Asn Arg Glu Asn Phe His Tyr Gly Ser Val Val Thr
            180                 185                 190
Tyr Arg Cys Asn Pro Gly Ser Gly Gly Arg Lys Val Phe Glu Leu Val
        195                 200                 205
Gly Glu Pro Ser Ile Tyr Cys Thr Ser Asn Asp Asp Gln Val Gly Ile
    210                 215                 220
Trp Ser Gly Pro Ala Pro Gln Cys Ile Ile Pro Asn Lys Cys Thr Pro
225                 230                 235                 240
Pro Asn Val Glu Asn Gly Ile Leu Val Ser Asp Asn Arg Ser Leu Phe

                245                 250                 255
Ser Leu Asn Glu Val Val Glu Phe Arg Cys Gln Pro Gly Phe Val Met
            260                 265                 270
Lys Gly Pro Arg Arg Val Lys Cys Gln Ala Leu Asn Lys Trp Glu Pro
        275                 280                 285
Glu Leu Pro Ser Cys Ser Arg Val Cys Gln Pro Pro Pro Asp Val Leu
    290                 295                 300
His Ala Glu Arg Thr Gln Arg Asp Lys Asp Asn Phe Ser Pro Gly Gln
305                 310                 315                 320
Glu Val Phe Tyr Ser Cys Glu Pro Gly Tyr Asp Leu Arg Gly Ala Ala
                325                 330                 335
Ser Met Arg Cys Thr Pro Gln Gly Asp Trp Ser Pro Ala Ala Pro Thr
            340                 345                 350
Cys Glu Val Lys Ser Cys Asp Asp Phe Met Gly Gln Leu Leu Asn Gly
        355                 360                 365
Arg Val Leu Phe Pro Val Asn Leu Gln Leu Gly Ala Lys Val Asp Phe
    370                 375                 380
Val Cys Asp Glu Gly Phe Gln Leu Lys Gly Ser Ser Ala Ser Tyr Cys
385                 390                 395                 400
Val Leu Ala Gly Met Glu Ser Leu Trp Asn Ser Ser Val Pro Val Cys
                405                 410                 415
Glu Gln Ile Phe Cys Pro Ser Pro Pro Val Ile Pro Asn Gly Arg His
            420                 425                 430
Thr Gly Lys Pro Leu Glu Val Phe Pro Phe Gly Lys Thr Val Asn Tyr
        435                 440                 445
Thr Cys Asp Pro His Pro Asp Arg Gly Thr Ser Phe Asp Leu Ile Gly
    450                 455                 460
Glu Ser Thr Ile Arg Cys Thr Ser Asp Pro Gln Gly Asn Gly Val Trp
465                 470                 475                 480
Ser Ser Pro Ala Pro Arg Cys Gly Ile Leu Gly His Cys Gln Ala Pro
                485                 490                 495
Asp His Phe Leu Phe Ala Lys Leu Lys Thr Gln Thr Asn Ala Ser Asp
            500                 505                 510
Phe Pro Ile Gly Thr Ser Leu Lys Tyr Glu Cys Arg Pro Glu Tyr Tyr
        515                 520                 525
Gly Arg Pro Phe Ser Ile Thr Cys Leu Asp Asn Leu Val Trp Ser Ser
    530                 535                 540
Pro Lys Asp Val Cys Lys Arg Lys Ser Cys Lys Thr Pro Pro Asp Pro
545                 550                 555                 560
Val Asn Gly Met Val His Val Ile Thr Asp Ile Gln Val Gly Ser Arg
                565                 570                 575
Ile Asn Tyr Ser Cys Thr Thr Gly His Arg Leu Ile Gly His Ser Ser
            580                 585                 590
Ala Glu Cys Ile Leu Ser Gly Asn Ala Ala His Trp Ser Thr Lys Pro
        595                 600                 605
Pro Ile Cys Gln Arg Ile Pro Cys Gly Leu Pro Pro Thr Ile Ala Asn
    610                 615                 620
Gly Asp Phe Ile Ser Thr Asn Arg Glu Asn Phe His Tyr Gly Ser Val
625                 630                 635                 640
Val Thr Tyr Arg Cys Asn Pro Gly Ser Gly Gly Arg Lys Val Phe Glu
                645                 650                 655
Leu Val Gly Glu Pro Ser Ile Tyr Cys Thr Ser Asn Asp Asp Gln Val
            660                 665                 670
Gly Ile Trp Ser Gly Pro Ala Pro Gln Cys Ile Ile Pro Asn Lys Cys
        675                 680                 685
Thr Pro Pro Asn Val Glu Asn Gly Ile Leu Val Ser Asp Asn Arg Ser
    690                 695                 700
Leu Phe Ser Leu Asn Glu Val Val Glu Phe Arg Cys Gln Pro Gly Phe
705                 710                 715                 720
Val Met Lys Gly Pro Arg Arg Val Lys Cys Gln Ala Leu Asn Lys Trp
                725                 730                 735
Glu Pro Glu Leu Pro Ser Cys Ser Arg Val Cys Gln Pro Pro Pro Asp
            740                 745                 750

Val Leu His Ala Glu Arg Thr Gln Arg Asp Lys Asp Asn Phe Ser Pro
        755                 760                 765
Gly Gln Glu Val Phe Tyr Ser Cys Glu Pro Gly Tyr Asp Leu Arg Gly
    770                 775                 780
Ala Ala Ser Met Arg Cys Thr Pro Gln Gly Asp Trp Ser Pro Ala Ala
785                 790                 795                 800
Pro Thr Cys Glu Val Lys Ser Cys Asp Asp Phe Met Gly Gln Leu Leu
                805                 810                 815
Asn Gly Arg Val Leu Phe Pro Val Asn Leu Gln Leu Gly Ala Lys Val
            820                 825                 830
Asp Phe Val Cys Asp Glu Gly Phe Gln Leu Lys Gly Ser Ser Ala Ser
        835                 840                 845
Tyr Cys Val Leu Ala Gly Met Glu Ser Leu Trp Asn Ser Ser Val Pro
    850                 855                 860
Val Cys Glu Gln Ile Phe Cys Pro Ser Pro Pro Val Ile Pro Asn Gly
865                 870                 875                 880
Arg His Thr Gly Lys Pro Leu Glu Val Phe Pro Phe Gly Lys Ala Val
                885                 890                 895
Asn Tyr Thr Cys Asp Pro His Pro Asp Arg Gly Thr Ser Phe Asp Leu
            900                 905                 910
Ile Gly Glu Ser Thr Ile Arg Cys Thr Ser Asp Pro Gln Gly Asn Gly
        915                 920                 925
Val Trp Ser Ser Pro Ala Pro Arg Cys Gly Ile Leu Gly His Cys Gln
    930                 935                 940
Ala Pro Asp His Phe Leu Phe Ala Lys Leu Lys Thr Gln Thr Asn Ala
945                 950                 955                 960
Ser Asp Phe Pro Ile Gly Thr Ser Leu Lys Tyr Glu Cys Arg Pro Glu
                965                 970                 975
Tyr Tyr Gly Arg Pro Phe Ser Ile Thr Cys Leu Asp Asn Leu Val Trp
            980                 985                 990
Ser Ser Pro Lys Asp Val Cys Lys Arg Lys Ser Cys Lys Thr Pro Pro
        995                 1000                1005
Asp Pro Val Asn Gly Met Val His Val Ile Thr Asp Ile Gln Val Gly
    1010                1015                1020
Ser Arg Ile Asn Tyr Ser Cys Thr Thr Gly His Arg Leu Ile Gly His
1025                1030                1035                1040
Ser Ser Ala Glu Cys Ile Leu Ser Gly Asn Thr Ala His Trp Ser Thr
                1045                1050                1055
Lys Pro Pro Ile Cys Gln Arg Ile Pro Cys Gly Leu Pro Pro Thr Ile
            1060                1065                1070
Ala Asn Gly Asp Phe Ile Ser Thr Asn Arg Glu Asn Phe His Tyr Gly
        1075                1080                1085
Ser Val Val Thr Tyr Arg Cys Asn Leu Gly Ser Arg Gly Arg Lys Val
    1090                1095                1100
Phe Glu Leu Val Gly Glu Pro Ser Ile Tyr Cys Thr Ser Asn Asp Asp
1105                1110                1115                1120
Gln Val Gly Ile Trp Ser Gly Pro Ala Pro Gln Cys Ile Ile Pro Asn
                1125                1130                1135
Lys Cys Thr Pro Pro Asn Val Glu Asn Gly Ile Leu Val Ser Asp Asn
            1140                1145                1150
Arg Ser Leu Phe Ser Leu Asn Glu Val Val Glu Phe Arg Cys Gln Pro
        1155                1160                1165
Gly Phe Val Met Lys Gly Pro Arg Arg Val Lys Cys Gln Ala Leu Asn
    1170                1175                1180
Lys Trp Glu Pro Glu Leu Pro Ser Cys Ser Arg Val Cys Gln Pro Pro
1185                1190                1195                1200
Pro Glu Ile Leu His Gly Glu His Thr Pro Ser His Gln Asp Asn Phe
                1205                1210                1215
Ser Pro Gly Gln Glu Val Phe Tyr Ser Cys Glu Pro Gly Tyr Asp Leu
            1220                1225                1230
Arg Gly Ala Ala Ser Leu His Cys Thr Pro Gln Gly Asp Trp Ser Pro
        1235                1240                1245
Glu Ala Pro Arg Cys Ala Val Lys Ser Cys Asp Asp Phe Leu Gly Gln

1250                    1255                    1260
Leu Pro His Gly Arg Val Leu Phe Pro Leu Asn Leu Gln Leu Gly Ala
1265                    1270                    1275                    1280
Lys Val Ser Phe Val Cys Asp Glu Gly Phe Arg Leu Lys Gly Ser Ser
                1285                    1290                    1295
Val Ser His Cys Val Leu Val Gly Met Arg Ser Leu Trp Asn Asn Ser
            1300                    1305                    1310
Val Pro Val Cys Glu His Ile Phe Cys Pro Asn Pro Pro Ala Ile Leu
        1315                    1320                    1325
Asn Gly Arg His Thr Gly Thr Pro Ser Gly Asp Ile Pro Tyr Gly Lys
    1330                    1335                    1340
Glu Ile Ser Tyr Thr Cys Asp Pro His Pro Asp Arg Gly Met Thr Phe
1345                    1350                    1355                    1360
Asn Leu Ile Gly Glu Ser Thr Ile Arg Cys Thr Ser Asp Pro His Gly
                1365                    1370                    1375
Asn Gly Val Trp Ser Ser Pro Ala Pro Arg Cys Glu Leu Ser Val Arg
            1380                    1385                    1390
Ala Gly His Cys Lys Thr Pro Glu Gln Phe Pro Phe Ala Ser Pro Thr
        1395                    1400                    1405
Ile Pro Ile Asn Asp Phe Glu Phe Pro Val Gly Thr Ser Leu Asn Tyr
    1410                    1415                    1420
Glu Cys Arg Pro Gly Tyr Phe Gly Lys Met Phe Ser Ile Ser Cys Leu
1425                    1430                    1435                    1440
Glu Asn Leu Val Trp Ser Ser Val Glu Asp Asn Cys Arg Arg Lys Ser
                1445                    1450                    1455
Cys Gly Pro Pro Pro Glu Pro Phe Asn Gly Met Val His Ile Asn Thr
            1460                    1465                    1470
Asp Thr Gln Phe Gly Ser Thr Val Asn Tyr Ser Cys Asn Glu Gly Phe
        1475                    1480                    1485
Arg Leu Ile Gly Ser Pro Ser Thr Thr Cys Leu Val Ser Gly Asn Asn
    1490                    1495                    1500
Val Thr Trp Asp Lys Lys Ala Pro Ile Cys Glu Ile Ile Ser Cys Glu
1505                    1510                    1515                    1520
Pro Pro Pro Thr Ile Ser Asn Gly Asp Phe Tyr Ser Asn Asn Arg Thr
                1525                    1530                    1535
Ser Phe His Asn Gly Thr Val Val Thr Tyr Gln Cys His Thr Gly Pro
            1540                    1545                    1550
Asp Gly Glu Gln Leu Phe Glu Leu Val Gly Glu Arg Ser Ile Tyr Cys
        1555                    1560                    1565
Thr Ser Lys Asp Asp Gln Val Gly Val Trp Ser Ser Pro Pro Pro Arg
    1570                    1575                    1580
Cys Ile Ser Thr Asn Lys Cys Thr Ala Pro Glu Val Glu Asn Ala Ile
1585                    1590                    1595                    1600
Arg Val Pro Gly Asn Arg Ser Phe Phe Ser Leu Thr Glu Ile Ile Arg
                1605                    1610                    1615
Phe Arg Cys Gln Pro Gly Phe Val Met Val Gly Ser His Thr Val Gln
            1620                    1625                    1630
Cys Gln Thr Asn Gly Arg Trp Gly Pro Lys Leu Pro His Cys Ser Arg
        1635                    1640                    1645
Val Cys Gln Pro Pro Pro Glu Ile Leu His Gly Glu His Thr Leu Ser
    1650                    1655                    1660
His Gln Asp Asn Phe Ser Pro Gly Gln Glu Val Phe Tyr Ser Cys Glu
1665                    1670                    1675                    1680
Pro Ser Tyr Asp Leu Arg Gly Ala Ala Ser Leu His Cys Thr Pro Gln
                1685                    1690                    1695
Gly Asp Trp Ser Pro Glu Ala Pro Arg Cys Thr Val Lys Ser Cys Asp
            1700                    1705                    1710
Asp Phe Leu Gly Gln Leu Pro His Gly Arg Val Leu Leu Pro Leu Asn
        1715                    1720                    1725
Leu Gln Leu Gly Ala Lys Val Ser Phe Val Cys Asp Glu Gly Phe Arg
    1730                    1735                    1740
Leu Lys Gly Arg Ser Ala Ser His Cys Val Leu Ala Gly Met Lys Ala
1745                    1750                    1755                    1760

Leu Trp Asn Ser Ser Val Pro Val Cys Glu Gln Ile Phe Cys Pro Asn
                1765                1770                1775
Pro Pro Ala Ile Leu Asn Gly Arg His Thr Gly Thr Pro Phe Gly Asp
            1780                1785                1790
Ile Pro Tyr Gly Lys Glu Ile Ser Tyr Ala Cys Asp Thr His Pro Asp
        1795                1800                1805
Arg Gly Met Thr Phe Asn Leu Ile Gly Glu Ser Ser Ile Arg Cys Thr
    1810                1815                1820
Ser Asp Pro Gln Gly Asn Gly Val Trp Ser Ser Pro Ala Pro Arg Cys
1825                1830                1835                1840
Glu Leu Ser Val Pro Ala Ala Cys Pro His Pro Pro Lys Ile Gln Asn
                1845                1850                1855
Gly His Tyr Ile Gly Gly His Val Ser Leu Tyr Leu Pro Gly Met Thr
            1860                1865                1870
Ile Ser Tyr Ile Cys Asp Pro Gly Tyr Leu Leu Val Gly Lys Gly Phe
        1875                1880                1885
Ile Phe Cys Thr Asp Gln Gly Ile Trp Ser Gln Leu Asp His Tyr Cys
    1890                1895                1900
Lys Glu Val Asn Cys Ser Phe Pro Leu Phe Met Asn Gly Ile Ser Lys
1905                1910                1915                1920
Glu Leu Glu Met Lys Lys Val Tyr His Tyr Gly Asp Tyr Val Thr Leu
                1925                1930                1935
Lys Cys Glu Asp Gly Tyr Thr Leu Glu Gly Ser Pro Trp Ser Gln Cys
            1940                1945                1950
Gln Ala Asp Asp Arg Trp Asp Pro Pro Leu Ala Lys Cys Thr Ser Arg
        1955                1960                1965
Thr His Asp Ala Leu Ile Val Gly Thr Leu Ser Gly Thr Ile Phe Phe
    1970                1975                1980
Ile Leu Leu Ile Ile Phe Leu Ser Trp Ile Ile Leu Lys His Arg Lys
1985                1990                1995                2000
Gly Asn Asn Ala His Glu Asn Pro Lys Glu Val Ala Ile His Leu His
                2005                2010                2015
Ser Gln Gly Gly Ser Ser Val His Pro Arg Thr Leu Gln Thr Asn Glu
            2020                2025                2030
Glu Asn Ser Arg Val Leu Pro
        2035

<210> 2
<211> 64
<212> DNA
<213> Artificial Sequence

<220>
<223> sequencing result

<400> 2
actgtccttt ccacagtgtg tcagccgcct ccagaaatcc tgcatggtga gcrtacccca      60

agcc                                                                    64

<210> 3
<211> 64
<212> DNA
<213> Artificial Sequence

<220>
<223> sequencing result

<400> 3
actgtccttt ccacagtgtg tcagccgcct ccagaaatcc tgcatggtga gcrtacccca      60

agcc                                                                    64

<210> 4
<211> 64
<212> DNA
<213> Artificial Sequence

<220>
<223> sequencing result

<400> 4
actgtccttt ccacagtgtg tcagccgcct ccagaaatcc tgcatggtga gcatacccca      60

agcc                                                                    64

<210> 5
<211> 64
<212> DNA
<213> Artificial Sequence

<220>
<223> sequencing result

<400> 5
actgtccttt ccacagtgtg tcagccgcct ccagaaatcc tgcatggtga gcatacccca      60

agcc                                                                    64

<210> 6
<211> 64
<212> DNA
<213> Artificial Sequence

<220>
<223> sequencing result

<400> 6
actgtccttt ccacagtgtg tcagccgcct ccagaaatcc tgcatggtga gcrtacccca      60

agcc                                                                    64